# EUROPEAN PATENT APPLICATION

(11) **EP 0 535 256 A1**
(43) Date of publication of application: **07.04.1993**
(21) Application number: 91114635.5
(22) Date of filing: 30.08.1991
(51) Int. Cl.: C07C 233/78, A61K 31/165, C07D 295/125, A61K 31/445

(54) **Substituted 1-phenyl-1-benzoylamino-5-aminopentanes, their preparation and use**

(71) Applicant: ZENTRUM FÜR CHEMIE DER ARZNEIMITTEL, 119815 Moskau (SU); WISSENSCHAFTLICHES ALLUNIONSZENTRUM FÜR GEFAHRLOSIGKEIT DER BIOLOGISCH WIRKSAMEN SUBSTANZEN, Noginski Bezirk, 142450 Moskauer Gebiet (SU); KARDIOLOGISCHES WISSENSCHAFTLICHES ALLUNIONSZENTRUM, 121552 Moskau (SU)
(72) Inventor: Mashkovsky, Mikhail Davidovich, 125057 Moskau (SU); Glushkov, Robert Georgievich, 125047 Moskau (SU); Skachilova, Sofiya Yakovlevna, 142450 Staraya Kupavna Moskauer Gebiet (SU); Dorodnikova, Elena Vladimirovna, 117418 Moskau (SU); Rosenshtraukh, Leonid Valentinovich, 121609 Moskau (SU); Voronin, Vasily Grigorevich, 142450 Staraya Kupavna Moskauer Gebiet (SU); Zheltukhin, Nikolai Konstantinovich, 142450 Staraya Kupavna Moskauer Gebiet (SU); Anjukhovsky, Evgenii Pavlovich, 121609 Moskau (SU); Nesterenko, Vladislav Vladislavovich, 121609 Moskau (SU); Cherkasova, Elena Mikhailovna, 103001 Moskau (SU); Zaitseva, Klara Alekseevna, 109457 Moskau (SU)
(74) Representative: Patentanwälte Zellentin & Partner

(57) **Abstract**

Abstract of the disclosure: 1-Phenyl-1-benzoylamino-5-aminopentanes, their optical isomers, their salts and the preparation thereof are described. The compounds are suitable for controlling diseases.

## Description

The present invention relates to substituted 1-phenyl-1-benzoylamino-5-aminopentanes, their preparation and use as drugs exhibiting antiarrhythmic and antifibrillatory action.

The search for novel agents intended to treat rhythm disturbances is currently an urgent problem.

Rhythm disturbance often complicates the course of various cardiovascular diseases. Arrhythmias are especially common among patients suffering from chronic ischemic heart disease. Thus, in 80-90% of cases arrhythmias complicate the course of acute myocardial infarction and are one of the main reasons of a fatal outcome.

Known in the art are various antiarrhythmic agents, for example Novocainamide (procainamide hydrochloride - β-diethylaminoethylamide of p-aminobenzoic acid hydrochloride) or disopyramide (4-diisopropylamino-2-phenyl-2-(2-pyridiyl)-butyramide phosphate) [M.D. Mashkovsky, Medicinal Agents, 1984, Meditsina (Moscow), pp. 403, 406; Martindale. The Extra Pharmacopoeia, 28th ed. London 1982, pp. 1375-1378].

Novocainamide has a series of side effects and thus its use in clinical practice is limited. Disopyramide is more active and is better tolerated by patients. However it also has some side effects, such as reduction in myocardial contractility, hypotension, atony of the intestine and urinary bladder and others.

Also known in the art is amiodarone (2-butyl-3-benzofuranyl-4-(2-diethylaminoethoxy)-3,5-diidophenyl ketone hydrochloride) which has antiarrhythmic and antianginal actions and is widely used for the prophylaxis and treatment of rhythm disturbances. [Martindale. The Extra Pharmacopoeia, 28th ed. London 1982, pp 1374-1375; M.D. Mashkovsky, Medicinal Agents, 1984, Meditsina, M., vol. 1, p. 425, Am. Heart J. 109 (1985) 949, J. Clin. Pharmacol. 27 (1987) 708).

This drug however exerts unwanted side effects related to the presence of iodine in its molecule. Among these are photo-dermatitis, keratitis and allergic reactions.

Also known in the art is quinidine (sulfate of (+) isomer of 5-vinyl-2-quinuclidyl-(6-methoxy-4-quinolyl)methanol) which has an antiarrhythmic action and is used for the treatment of paroxysmal tachycardias, atrial fibrillation and extrasystoles. However in cases of overdosage and heightened individual sensitivity quinidine reduced cardiac activity and has a number of side effects (allergic reactions, diarrhea, nausea and others). [Martindale. The Extra Pharmacopoeia, 28th ed. London 1982, pp. 1370-1373, M.D. Mashkovsky, Medicinal Agents, 1984, Meditsina, M., vol. 1, p. 351].

It is further known that some 1-phenyl-1-benzoylamino-5-aminopentanes have an antiarrhythmic action [Khimiko-Farmatserticheskii Zhurnal 15 (1981) 43, Doklady Akademii Nauk. SSSR 313 (1990) 616].

The present invention is aimed at the development of novel compounds possessing a high antiarrhythmic and antifibrillatory activity and good tolerability of drugs based on these compounds.

The present invention relates to 1-phenyl-1-benzoylamino-5-aminopentanes and their optically active isomers, having the formula I: 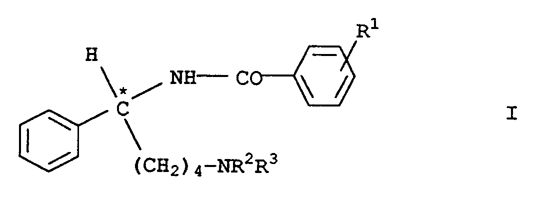
where
- R¹: is a halogen atom, a nitro group, a C₁₋₄-aminoacyl group, or a sulfonamido group,
- R²: and R³ are C₁₋₅-alkyl groups or together form a C₃₋₆-alkylene group
and their salts with physiologically tolerated acids.
The compounds according to the invention are obtained by
1. acylating 1-phenyl-1-amino-5-aminopentanes of the formula II 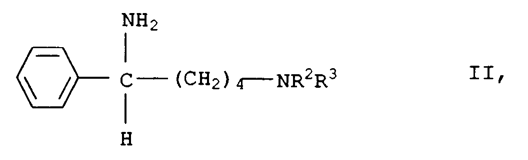 where R² and R³ are as identified above, with a benzoyl halide of the formula III 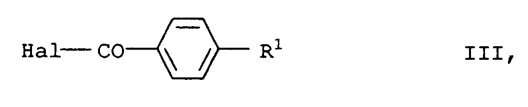 where R¹ is as identified above, or
2. reacting a compound of the formula IV 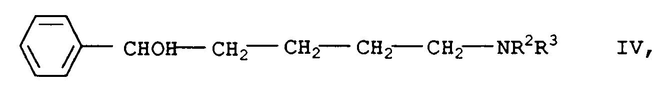 where R² and R³ are as identified above, with a nitrile of the formula V 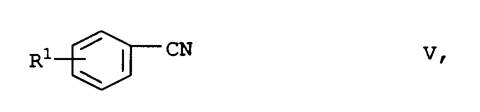 where R¹ is as identified above,
and, if desired, subsequently separating the resulting compound into its optically active antipodes and/or converting it into a salt thereof with a physiologically tolerated acid.

In the case of process 1.), the reaction is preferably carried out in the presence of an aprotic diluent or solvent, for example an ether, ketone, or acetonitrile, and expediently at temperatures between 0°C and the boiling point of the solvent used.

The preferred solvent for the reaction of the compounds II and III is methyl-t-butyl ether, the reaction preferably being carried out between 0°C and 45°C.

The reaction time depends on the reactants employed; in general, the reaction is complete after from 2 to 20 hours.

The reaction product can be obtained in a conventional manner, eg. by filtration or removal of the diluent or solvent from the reaction mixture by distillation. The resulting compound is purified in a conventional manner, for example by recrystallization from a solvent.

The starting compounds of the general formula II are known or can be prepared by methods known from the literature and as described, for example, in Houben-Weyl, Methoden der organischen Chemie (Methods in Organic Chemistry), Vol. 11/1, pages 611 et seq., G. Thieme Verlag, Stuttgart 1957, by catalytic hydrogenation in the presence of ammonia or by reacting the corresponding aminoketones with ammonium formate.

The reaction of compounds of the general formula IV with a nitrile V in process 2.) can be carried out at 0°C and 25°C. This reaction is expediently carried out with the addition of a mineral acid. The preferred mineral acid is sulfuric acid at a concentration of between 60 and 90 per cent.

The reaction product can be obtained in a conventional manner, eg. by diluting the reaction mixture, followed by filtration or extraction. The resulting compound is purified in a conventional manner, for example by recrystallisation from a solvent. The subsequent conversion into an acid addition compound, is carried out in a conventional manner.

The starting compounds IV can be prepared according to methods known from the literature, eg., by catalytic hydrogenation of the corresponding ketones or by reducing these ketones with complex hydrides.

The compounds of the general formula V are known or can be prepared according to methods known in the art.

It is possible to convert the resulting compounds according to the invention into the acid addition salts thereof with physiologically tolerated acids. Examples of suitable conventional physiologically tolerated organic or inorganic acids are hydrochloric acid, oxalic acid, maleic acid, fumaric acid, lactic acid, tartaric acid, adipic acid or benzoic acid. Others can be found in Fortschritte der Arzneimittelforschung, Vol. 10, pages 224 et seq., Birkhauser Verlag, Basel und Stuttgart, 1966. The hydrochloride is preferred.

The acid addition salts are, as a rule, obtained in a conventional manner by mixing the free base or solutions thereof with the appropriate acid or solutions thereof in an organic solvent, for example a lower alcohol such as methanol, ethanol or propanol, or an ether such as diethyl ether or methyl t-butylether. It is also possible, to improve deposition of crystals, to use mixtures of the said solvents. Furthermore, pharmaceutically acceptable aqueous solutions of acid addition compounds of the compounds I according to the invention can be prepared by dissolving the free bases in an aqueous acid solution.

The compounds of the formula I according to the invention have a center of chirality and are obtained as racemates which can be separated by conventional methods, for example by forming diastereomeric salts with optically active acids, into the optically active antipodes.

Racemic 1-phenyl-1-p-nitrobenzoylamino-5-N,N-diethylaminopentane is separated into optically active isomers using eg. dibenzoyl-(+)-tartaric acid as asymmetric reagent in acetone and benzene, and the obtained tartrates are recrystallized from acetonitrile with the subsequent isolation of the enantiomers from the tartrates in the form of bases and their transformation into their salts.

Preferred compounds according to the present invention are 1-phenyl-1-p-nitrobenzoylamino-5-N,N-diethylaminopentane (compound 1a);
(-)1-phenyl-1-p-nitrobenzoylamino-5-N,N-diethylaminopentane (compound 1b);
(+)1-phenyl-1-p-nitrobenzoylamino-5-N,N-diethylaminopentane (compound 1c);
and especially their hydrochlorides, which are yellowish-wite crystalline substances readily soluble in water and ethyl alcohol.

The structure and stereochemistry of the compounds according to the present invention are confirmed by spectral data (IR, PMR, mass spectroscopy), as well as by elemental analysis.

The compounds according to the present invention can be used against arrhythmias. They are especially useful against reentry arrhythmias in atria and ventricles and against arrhythmias in connexion with cardiomyopathies and heart insufficiencies. They are of low toxicity and do not have a cardiotoxic effect. They are neither allergenic nor mutagenic.

The compounds according to the invention can be administered orally or, preferably, parenterally in a conventional manner.

The dosage depends on the age, condition and weight of the patient and on the mode of administration. As a rule, the daily dose of active substance is from 0.1 to 1.5 mg/kg of body weight on parenteral administration.

The novel compounds can be administered in conventional solid or liquid pharmaceutical forms, e.g. as uncoated or (film-) coated tablets, capsules, powders, granules or solutions. These are produced in a conventional manner. The active substances can be processed for this purpose with the conventional pharmaceutical auxiliaries such as tablet binders, fillers, preservatives, tablet disintegrants, flow regulators, plasticizers, wetting agents, dispersants, emulsifiers, solvents, retardants, antioxidants and/or propellant gases (cf. H. Sucker et al.: Pharmazeutische Technologie, Thieme-Verlag, Stuttgart, 1978). The forms obtained in this way normally contain from 0.1 to 90 percent by weight of the active substance.

The following examples illustrate the invention.

### Example 1

3.04 g (0.01 mol) of 1-phenyl-1-amino-5-N,N-diethylaminopentane dihydrochloride, 20 ml (0.04 mol) of a 10% aqueous solution of sodium hyroxide and 6 ml of acetone are placed in a three-neck flask equipped with a mechanical mixer and thermometer. The mixture is cooled to 0 - 5°C, and at this temperature 2 g (0.011) mol) of p-nitrobenzoyl chloride are added at the rate of 0.5 g every 5 min. Then the reaction mixture is stirred to form a homogeneous suspension. The precipitate is filtered off, washed in succession with 10 ml of a 2% aqueous solution of sodium hydroxide, 20 ml of water and 5 ml of ethyl ether and is air-dried at room temperature.

2.4 g (0.0062 mol) of 1-phenyl-1-p-nitrobenzoylamino-5-N,N-diethylaminopentane are obtained with melting point 99 - 101°C. The yield is 62%. To obtain the hyrochloride, the product is dissolved in acetone and acidified with HCl solution in isopropanol. The precipitate is filtered off. 2.45 g of 1-phenyl-1-p-nitrobenzoylamino-5-N,N-diethylaminopentane hydrochloride are obtained (m.p. 165 - 167°C).

| Elemental analysis of 1-phenyl-1-p-nitrobenzoylamino-5-N,N-diethylaminopentane hydrochloride : C₂₂H₃₀N₃O₃Cl | | | | |
|---|---|---|---|---|
| Calculated, %: | C 62.92; | H 7.14; | N 10.01; | Cl 8.44 |
| Found, % | C 62.48; | H 7.64; | N 9.67; | Cl 8.41. |

IR spectrum: ν CO 1636 cm⁻¹; ν C-C 1598 cm⁻¹. 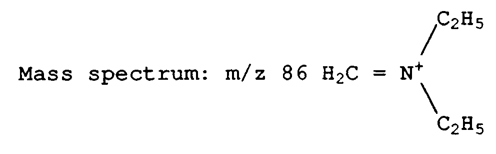
M⁺ 383 (corresponds to molecular mass 382 + 1);
PMR spectrum: (in D₂O) δ, ppm: protons CH₂ - N: 3.21-2.81;
p - C₆H₄NO₂ 8.32; 7.91; 8.8 H 5.0, triplet 7.2.
CH₂-CH₃ 1.28, triplet 7.3.
The following compounds were prepared in a similar manner:

### Example 2

1-Phenyl-1-p-nitrobenzoylamino-5-piperidin-1-ylpentane hyrochloride (m.p. 128-130°C)

### Example 3

1-Phenyl-1-p-bromobenzoylamino-5-piperidin-1-ylpentane hydrochloride (m.p. 101-103°C)

### Example 4

1-Phenyl-1-p-acetamidobenzoylamino-5-piperidin-1-ylpentane hydrochloride (m.p. 99-101°C)

### Example 5

1-Phenyl-1-p-nitrobenzoylamino-5-N,N-diethylaminopentane (racemate) is obtained in a manner similar to that described in Example 1 and is resolved into optically active isomers in the following way.

10 g (0.026 mol) of 1-phenyl-1-p-nitrobenzoylamino-5-N,N-diethylaminopentane in 100 ml (92.8 g) of actone are placed in a three-neck flask with mixer, reflux condenser and dropping funnel; a solution of 9.33 g (0.026 mol) of dibenzoyl-8+)-tartaric acid in 50 ml of acetone mixed with benzene (1:1 is added, and the reaction mixture is stirred for 1 h. The precipitate is filtered off. The yield is 8.9 g (92%) of (+)-tartrate of 1-phenyl-1-p-nitrobenzoylamino-(+) 5-diethylaminopentane, with melting point 136-137°C and [α]_{D}²⁰ = 14.3° (2% in dimethylformamie). The mother liquor is evaporated in vacuo, the residue is washed with acetone, and the yield is 9.0 g (95%) of (+)-tartrate of 1-phenyl-1-p-nitrobenzoylamino-(-)-5-diethylaminopentane, with melting point 135-136°C and [α]_{D}²⁰ = -40.6° (2%, dimethylformamide).

The obtained diastereomeric salts are decomposed with an aqueous solution of ammonia, extracted with ethyl acetate and, after distillation of the latter, 4.45 g (96.5%) of (-) enantiomer calculated as tartrate are obtained, with melting point 102-104°C and [α]_{D}²⁰ = -31.85° (3%, acetonitrile).

The obtained enantiomers are dissolved in isopropyl alcohol, HCl solution in alcohol is added to pH 4, the solvent is distilled off, the residue is recrystallised from acetone and the yield is 4.68 g (95%) of hyrochloride of (+)-enantiomer, with melting point 155-165°C, [α]_{D}²⁰ = +31.6° (3%, dimethylformamide), +13.2° (1%, water). C₂₂H₃₀N₃O₃Cl

| | | | | |
|---|---|---|---|---|
| Calculated %: | C 62.92; | H 7.42; | N 10.01; | Cl 8.44 |
| Found %: | C 62.78, | H 7.53, | N 9.78, | Cl 8.45; |

4.09 g (92%) of hydrochloride of (-) enantiomer with melting point 155-165°C, [α]_{D}²⁰ = -30.6°C (3%, dimethylformamide), -13.8 (1%, water). C₂₂H₃₀N₃O₃Cl

| | | | | |
|---|---|---|---|---|
| Calculated %: | C 62.92; | H 7.42; | N 10.01; | Cl 8.44 |
| Found % : | C 62.70; | H 7.57; | N 9.76; | Cl 8.46. |

## Claims

1. 1-Phenyl-1-benzoylamino-5-aminopentanes and their optically active isomers, having the formula I: 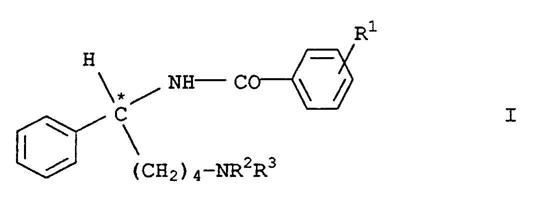 where
R¹ is a halogen atom, a nitro group, a C₁₋₄-aminoacyl group, or a sulfonamido group,
R² and R³ are C₁₋₅-alkyl groups or together form a C₃₋₆-alkylene group
and their salts with physiologically tolerated acids.

2. (∓)1-Phenyl-1-p-nitrobenzoylamino-5-N,N-diethylaminopentane hydrochloride.

3. (-)1-Phenyl-1-p-nitrobenzoylamino-5-N,N-diethylaminopentane hydrochloride.

4. (+)1-Phenyl-1-p-nitrobenzoylamino-5-N,N-diethylaminopentane hydrochloride.

5. A drug with antiarrhythmic and antifibrillatory activity which contains a compound as claimed in claims 1-4, and a pharmaceutical carrier.

6. The method for preparing 1-phenyl-1-benzoylamino-5-diethylaminopentanes of the formula I according to claim 1 characterized by
1. acylating 1-phenyl-1-amino-5-aminopentanes of the formula II 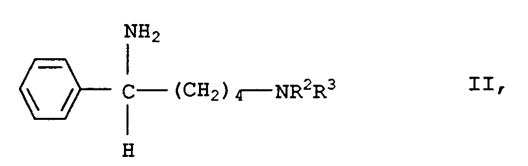 where R² and R³ are as identified above, with a benzoyl halide of the formula III 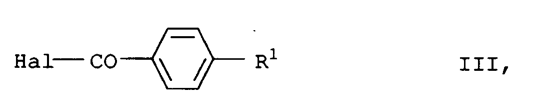 where R¹ is as identified above, or
2. reacting a compound of the formula IV 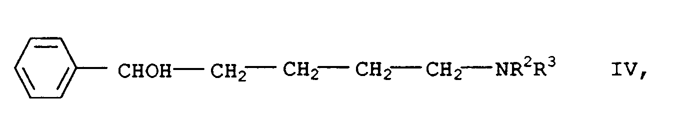 where R² and R³ are as identified above, with a nitrile of the formula V 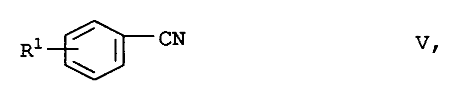 where R¹ is as identified above,
and, if desired, subsequently converting the resulting compound into its optically active antipodes and/or converting it into a salt thereof with a physiologically tolerated acid.
